# EUROPEAN PATENT APPLICATION

(11) **EP 3 037 531 A1**
(43) Date of publication of application: **29.06.2016**
(21) Application number: 14837506.6
(22) Date of filing: 20.08.2014
(51) Int. Cl.: C12N 15/09, C12Q 1/68, G01N 33/53

(54) **METHOD AND KIT FOR MEASURING MODIFIED NUCLEIC-ACID BASE USING HETEROGENEOUS NUCLEIC ACID PROBE**

(30) Priority: 21.08.2013 JP 2013171658
(71) Applicant: Fujirebio Inc., Shinjuku-ku Tokyo 163-0410 (JP)
(72) Inventor: MATSUNO, Tatsuki, Tokyo 163-0410 (JP); HORIIKE, Mariko, Tokyo 163-0410 (JP)
(74) Representative: Hoffmann Eitle
(86) International application number: PCT/JP2014/071703
(87) International publication number: WO 2015/025862

(57) **Abstract**

The present invention provides a technique that suppresses a background value of a detection signal to construct an immunoassay system that detects a modified nucleobase. Specifically, the present invention provides a method for measuring a modified nucleobase comprising incubating a nucleic acid sample (e.g., a sample containing a target DNA containing the modified nucleobase) and a heterogeneous nucleic acid probe (e.g., an RNA probe) in a solution and measuring the modified nucleobase using an antibody against the modified nucleobase in the obtained solution. The present invention also provides a kit for measuring a modified nucleobase comprising a heterogeneous nucleic acid probe and an antibody against the modified nucleobase.

SEQUENCE LISTING

<110>  FUJIREBIO INC.

<120>  Methods of measuring modified nucleobases with heterologous
       nucleic acid probe and kits therefor

<130>  PRBA-26632

<150>  JP2013-171658
<151>  2013-08-21

<160>  13

<170>  PatentIn version 3.5

<210>  1
<211>  27
<212>  DNA
<213>  Artificial Sequence

<220>
<223>  Nucleotide sequence of a certain target nucleic acid.  The 13th
       nucleotide (cytosine) is methylated at position 5.

<400>  1
ttgcgcggcg tccgtcctgt tgacttc                                          27


<210>  2
<211>  26
<212>  RNA
<213>  Artificial Sequence

<220>
<223>  Heterologous nucleic acid probe for capturing the target nucleic
       acid of SEQ ID NO:1 or 4.

<400>  2
gaagucaaca ggacgacgcc gcgcaa                                           26


<210>  3
<211>  26
<212>  DNA
<213>  Artificial Sequence

<220>
<223>  Homologous nucleic acid probe for capturing the target nucleic
       acid of SEQ ID NO:1 or 4.

<400>  3
gaagtcaaca ggacgacgcc gcgcaa                                           26


<210>  4
<211>  47
<212>  DNA
<213>  Artificial Sequence

<220>
<223>  Nucleotide sequence of a certain target nucleic acid.  The 7th
       nucleotide (cytosine) is methylated at position 5.

<400>  4
aatcagcggg agctctttct ttgcgcggcg tccgtcctgt tgacttc                    47


<210>  5
<211>  45
<212>  DNA
<213>  Artificial Sequence

<220>
<223>  Nucleotide sequence of a certain target nucleic acid.  The 2nd,
       15th and 20th nucleotides (cytosines) are methylated at position
       5.

<400>  5
gcggagctct ccctcgggac ggtggcagcc tcgagtggtc ctgca                      45


<210>  6
<211>  24
<212>  RNA
<213>  Artificial Sequence

<220>
<223>  Heterologous nucleic acid probe for capturing the target nucleic
       acid of SEQ ID NO:5.

<400>  6
ugcaggacca cucgaggcug ccac                                             24


<210>  7
<211>  24
<212>  DNA
<213>  Artificial Sequence

<220>
<223>  Homologous nucleic acid probe for capturing the target nucleic
       acid of SEQ ID NO:5.

<400>  7
tgcaggacca ctcgaggctg ccac                                             24


<210>  8
<211>  32
<212>  DNA
<213>  Artificial Sequence

<220>
<223>  Nucleotide sequence of a certain target nucleic acid.  The 2nd
       nucleotide (cytosine) is methylated at position 5.

<400>  8
tcgtggtgga cttctctcaa ttttctaggg gg                                    32


<210>  9
<211>  40
<212>  RNA
<213>  Artificial Sequence

<220>
<223>  Heterologous nucleic acid probe for capturing the target nucleic
      acid of SEQ ID NO:8.

<400>  9
cccccuagaa aauugagaga aguccaccac aaaaaaaaaa                          40


<210>  10
<211>  40
<212>  DNA
<213>  Artificial Sequence

<220>
<223>  Homologous nucleic acid probe for capturing the target nucleic
      acid of SEQ ID NO:8.

<400>  10
ccccctagaa aattgagaga agtccaccac aaaaaaaaa                          40


<210>  11
<211>  37
<212>  DNA
<213>  Artificial Sequence

<220>
<223>  Nucleotide sequence of a certain target nucleic acid.  The 35th
      nucleotide (cytosine) is methylated at position 5.

<400>  11
ggctcagttt actagtgcca tttgttcagt ggttcgt                            37


<210>  12
<211>  41
<212>  RNA
<213>  Artificial Sequence

<220>
<223>  Heterologous nucleic acid probe for capturing the target nucleic
      acid of SEQ ID NO:11.

<400>  12
cacugaacaa auggcacuag uaaacugagc caaaaaaaaa a                        41


<210>  13
<211>  41
<212>  DNA
<213>  Artificial Sequence

<220>
<223>  Homologous nucleic acid probe for capturing the target nucleic
      acid of SEQ ID NO:11.

<400>  13
cactgaacaa atggcactag taaactgagc caaaaaaaaa a                        41

## Description

### TECHNICAL FIELD

The present invention relates to a method and a kit for measuring a modified nucleobase.

### BACKGROUND ART

Many techniques that detect nucleobases to which substances such as biotin are artificially introduced by immunoassays using antibodies in nucleic acid (e.g., DNA, RNA) detection have been reported. Techniques that detect naturally occurring modified nucleobases (e.g., methylcytosine, hydroxymethylcytosine) by immunoassays are also reported (Patent Literature 1, and Non Patent Literature 1 and 2).

### Prior Art Reference

### Patent Literature

Patent Literature 1: Japanese Patent Application Laid-open No. 2012-230019

### Non-Patent Literature

Non Patent Literature 1: Proll et al., DNA Research, 13, 37-42 (2006)
Non Patent Literature 2: Kurita et al., Anal. Chem., 2012, 84, 7533-7538

### SUMMARY OF INVENTION

### PROBLEM TO BE SOLVED BY THE INVENTION

The inventors of the present invention have found out that in constructing the above immunoassay system that detects modified nucleobases, there is a problem in that a background value of a detection signal increases owing to non-specific binding of an antibody against the modified nucleobase to a homogeneous nucleic acid probe (a probe composed of a nucleic acid homogeneous with respect to a target nucleic acid). Consequently, a technique for suppressing the background value has been required to be developed in order to construct a high-sensitivity immunoassay system.

### MEANS FOR SOLVING PROBLEM

As a result of intensive investigations, the inventors of the present invention have succeeded in suppressing the background value and the like in the measurement of a modified nucleobase in a target nucleic acid using an antibody against the modified nucleobase by using a heterogeneous nucleic acid probe (a probe composed of a nucleic acid heterogeneous with respect to the target nucleic acid) in place of a homogeneous nucleic acid probe and have achieved the present invention.

That is, the present invention is as follows.
[1] A method for measuring a modified nucleobase, the method comprising:
   (1) incubating a nucleic acid sample and a heterogeneous nucleic acid probe in a solution; and
   (2) measuring the modified nucleobase using an antibody against the modified nucleobase in the solution obtained at (1).
[2] The method according to claim 1, wherein the nucleic acid sample contains a target nucleic acid containing the modified nucleobase, and the steps (1) and (2) are performed by (1') and (2'), respectively:
   (1') reacting the nucleic acid sample containing the target nucleic acid containing the modified nucleobase and the heterogeneous nucleic acid probe in the solution by incubation to form a heterogeneous nucleic acid hybrid composed of the target nucleic acid and the heterogeneous nucleic acid probe; and
   (2') measuring the modified nucleobase using the antibody against the modified nucleobase in a solution containing the heterogeneous nucleic acid hybrid.
[3] The method according to [1] or [2], wherein the target nucleic acid is a target nucleic acid potentially containing two or more modified nucleobases.
[4] The method according to any one of [1] to [3], further comprising adding the heterogeneous nucleic acid probe to a solution containing the nucleic acid sample to prepare a solution containing both the nucleic acid sample and the heterogeneous nucleic acid probe.
[5] The method according to any one of [1] to [4], wherein the nucleic acid sample is a sample containing a target DNA containing the modified nucleobase.
[6] The method according to any one of [1] to [5], wherein the heterogeneous nucleic acid probe is an RNA probe.
[7] The method according to any one of [1] to [6], wherein a nucleobase that composes the modified nucleobase is cytosine.
[8] The method according to any one of [1] to [7], wherein the modified nucleobase is methylcytosine.
[9] The method according to any one of [2] to [8], wherein the heterogeneous nucleic acid probe is designed such that an unpaired part of the modified nucleobase is formed in a double-stranded structure part of the heterogeneous nucleic acid hybrid.
[10] The method according to any one of [2] to [9], wherein the heterogeneous nucleic acid probe is designed such that the modified nucleobase is present in a single-stranded structure part of the heterogeneous nucleic acid hybrid.
[11] The method according to any one of [1] to [10], wherein the measurement of the modified nucleobase using the antibody against the modified nucleobase is performed by ELISA.
[12] A kit for measuring a modified nucleobase, the kit comprising:
   (I) a heterogeneous nucleic acid probe; and
   (II) an antibody against the modified nucleobase.

### EFFECT OF THE INVENTION

The method and the kit of the present invention can measure a modified nucleobase in a target nucleic acid with high sensitivity by reducing a background value of a detection signal.

### BRIEF DESCRIPTION OF DRAWINGS

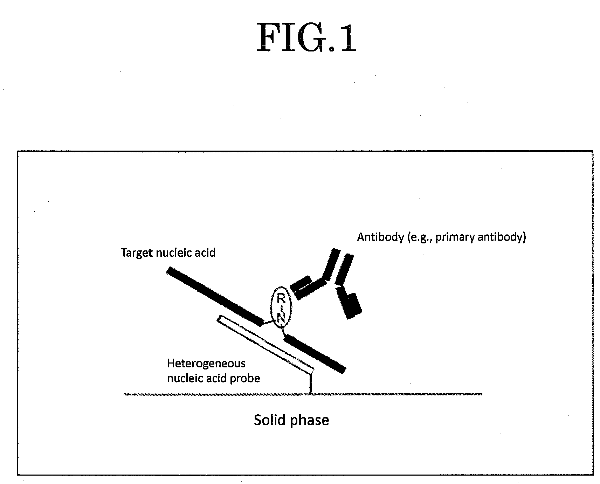

FIG. 1 is a diagram of an example of a measurement summary of a modified nucleobase in a target nucleic acid by the method of the present invention. R-N: a nucleotide residue having a modified nucleobase; N: a nucleotide residue having a nucleobase; and R: a substituent that a nucleobase has.

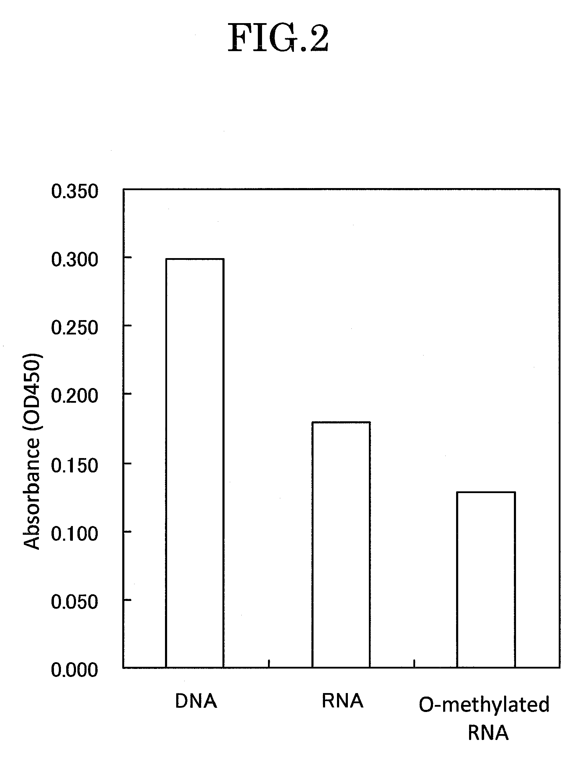

FIG. 2 is a diagram of signal values (background values) measured in measurement on the conditions of a target nucleic acid containing a modified nucleobase (-), a heterogeneous or homogeneous nucleic acid probe (+), and an antibody against the modified nucleobase (+). The target nucleic acid: DNA; and the nucleic acid probe: the heterogeneous nucleic acid probe (normal RNA or 2'-O-methylated RNA) or the homogeneous nucleic acid probe (DNA).

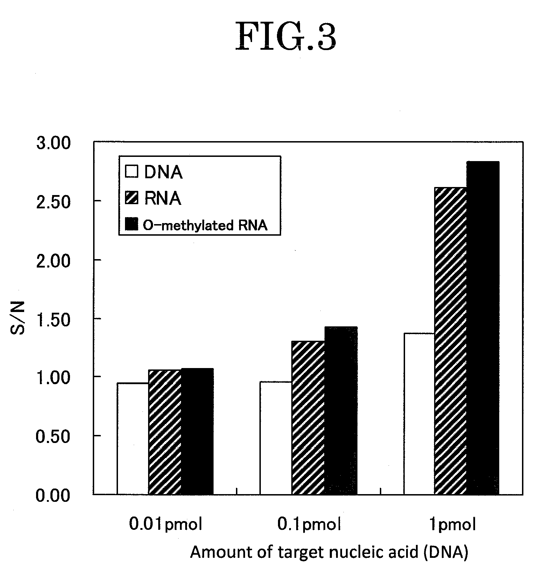

FIG. 3 is a diagram of signal-to-noise ratios (S/N) calculated in the measurement of the modified nucleobase of each of the concentrations using the nucleic acid probe. The used target nucleic acid and nucleic acid probe are similar to those in FIG. 2. In S/N, S indicates a signal value measured in measurement on the conditions of the target nucleic acid (+), the nucleic acid probe (+), and the antibody against the modified nucleobase (+), whereas N indicates a signal value (a background value) measured in measurement on the conditions of the target nucleic acid (-), the nucleic acid probe (+), and the antibody against the modified nucleobase (+).

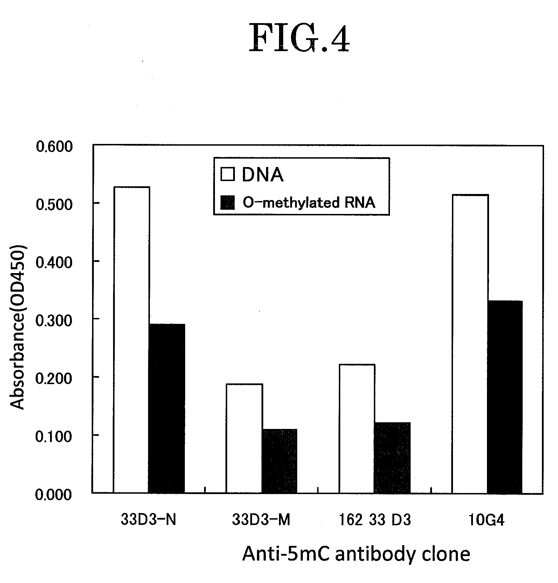

FIG. 4 is a diagram of signal values (background values) measured in measurement on the conditions of the target nucleic acid containing the modified nucleobase (-), the heterogeneous or homogeneous nucleic acid probe (+), and various types of antibodies against the modified nucleobase (+). The target nucleic acid: DNA; the nucleic acid probe: the heterogeneous nucleic acid probe (2'-O-methylated RNA) or the homogeneous nucleic acid probe (DNA). The used antibody clones are as follows: Clone33D3-N: an anti-methylcytosine antibody (manufactured by Nippon Gene Co., Ltd.); Clone33D3-M: an anti-methylcytosine antibody (manufactured by Millipore Corporation); Clone162 33 D3: an anti-methylcytosine antibody (manufactured by Millipore Corporation); and Clone10G4: an anti-methylcytosine antibody (manufactured by Zymo Research Corporation).

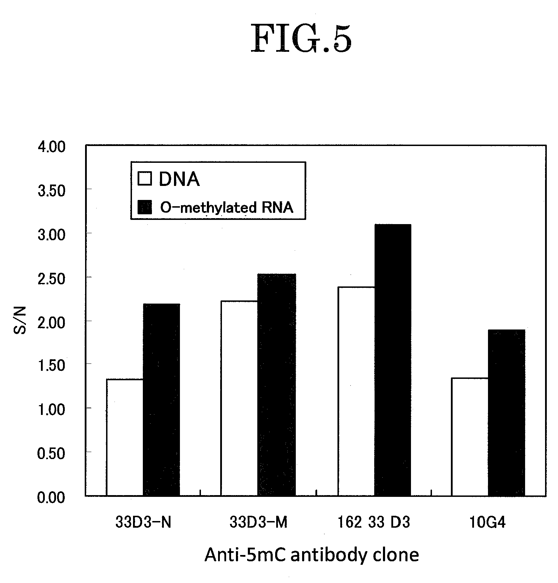

FIG. 5 is a diagram of signal-to-noise ratios (S/N) calculated in the measurement of the modified nucleobase using the various types of antibodies against the modified nucleobase and the nucleic acid probe. The used target nucleic acid, nucleic acid probe, and antibody clones are similar to those in FIG. 4. S/N is similar to that in FIG. 3.

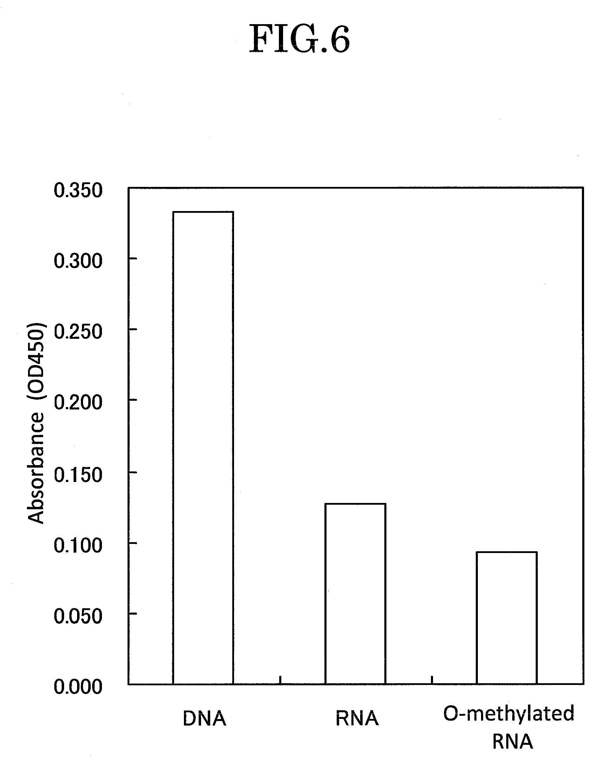

FIG. 6 is a diagram of signal values (background values) measured in measurement on the conditions of the target nucleic acid containing the modified nucleobase (-), the heterogeneous or homogeneous nucleic acid probe (+), and the antibody against the modified nucleobase (+). The target nucleic acid: DNA; the nucleic acid probe: the heterogeneous nucleic acid probe (normal RNA or 2'-O-methylated RNA) or the homogeneous nucleic acid probe (DNA).

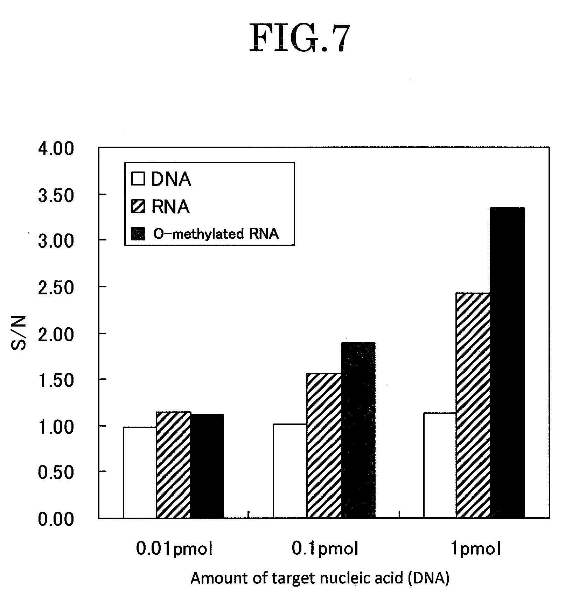

FIG. 7 is a diagram of signal-to-noise ratios (S/N) calculated in the measurement of the modified nucleobase of each of the concentrations using the nucleic acid probe. The used target nucleic acid and nucleic acid probe are similar to those in FIG. 6.

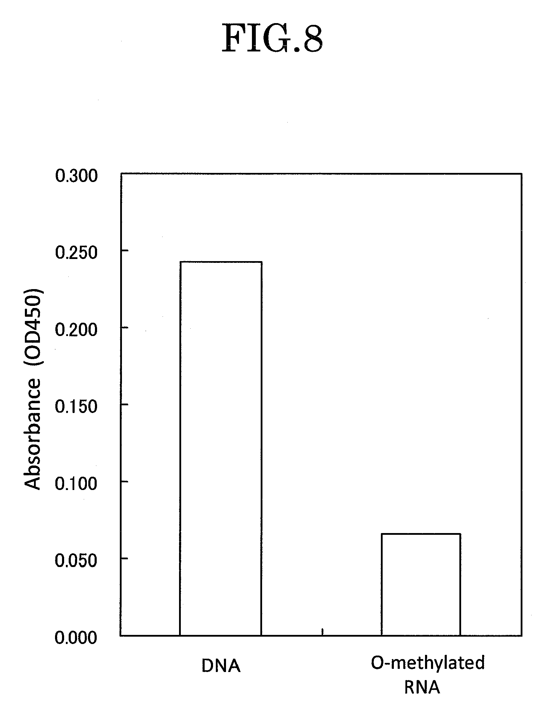

FIG. 8 is a diagram of signal values (background values) measured in measurement on the conditions of the target nucleic acid containing the modified nucleobase (-), the heterogeneous or homogeneous nucleic acid probe (+), and the antibody against the modified nucleobase (+). The target nucleic acid: DNA; the nucleic acid probe: the heterogeneous nucleic acid probe (2'-O-methylated RNA) or the homogeneous nucleic acid probe (DNA).

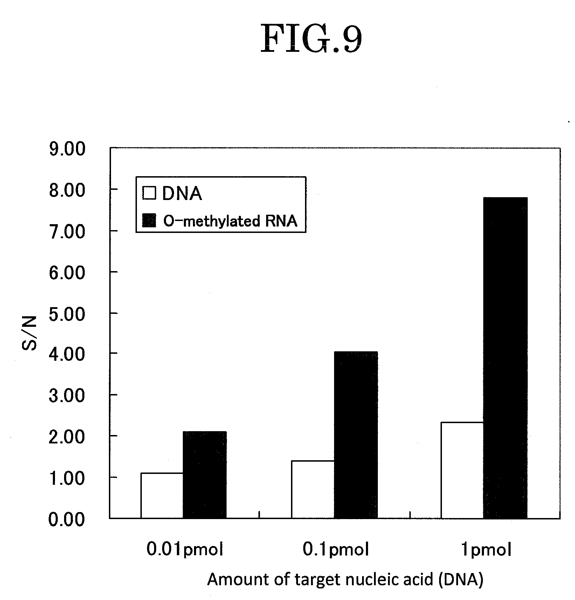

FIG. 9 is a diagram of signal-to-noise ratios (S/N) calculated in the measurement of the modified nucleobase of each of concentrations using the nucleic acid probe. The used target nucleic acid and nucleic acid probe are similar to those in FIG. 8.

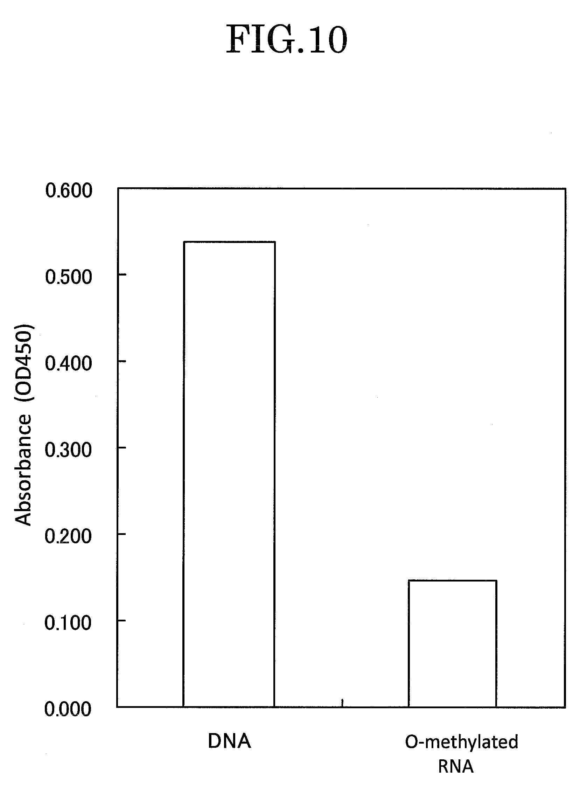

FIG. 10 is a diagram of signal values (background values) measured in measurement on the conditions of the target nucleic acid containing the modified nucleobase (-), the heterogeneous or homogeneous nucleic acid probe (+), and the antibody against the modified nucleobase (+). The target nucleic acid: DNA; the nucleic acid probe: the heterogeneous nucleic acid probe (2'-O-methylated RNA) or the homogeneous nucleic acid probe (DNA).

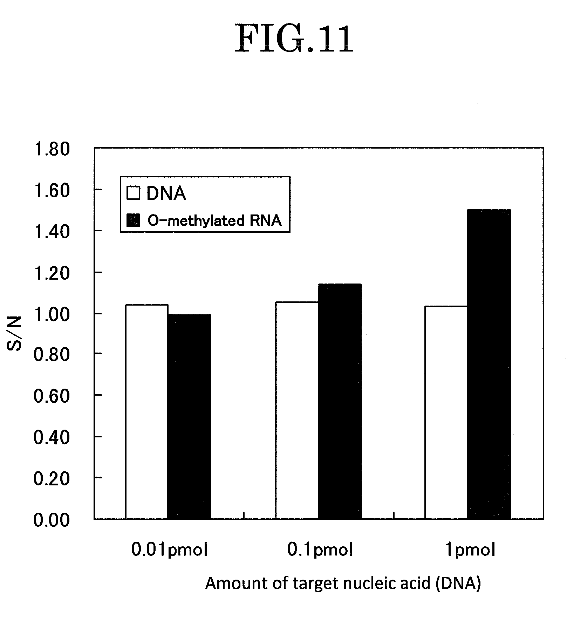

FIG. 11 is a diagram of signal-to-noise ratios (S/N) calculated in the measurement of the modified nucleobase of each of the concentrations using the nucleic acid probe. The used target nucleic acid and nucleic acid probe are similar to those in FIG. 10.

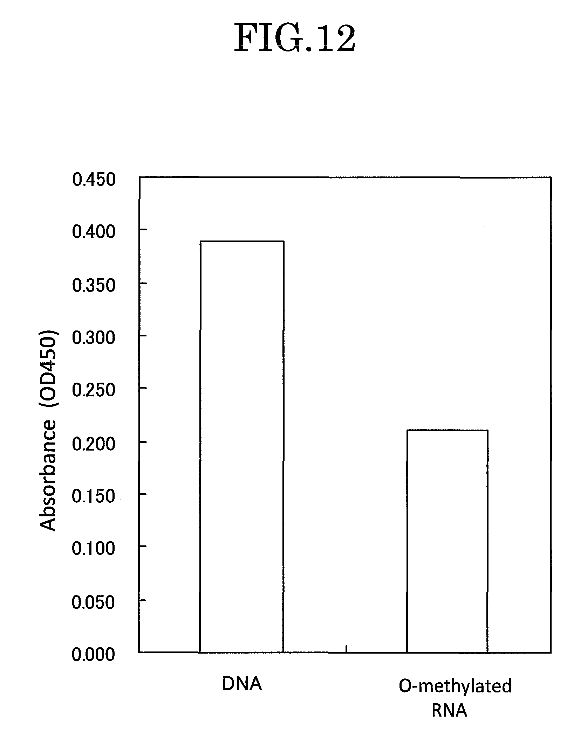

FIG. 12 is a diagram of signal values (background values) measured in measurement on the conditions of the target nucleic acid containing the modified nucleobase (-), the heterogeneous or homogeneous nucleic acid probe (+), and the antibody against the modified nucleobase (+). The target nucleic acid: DNA; the nucleic acid probe: the heterogeneous nucleic acid probe (2'-O-methylated RNA) or the homogeneous nucleic acid probe (DNA).

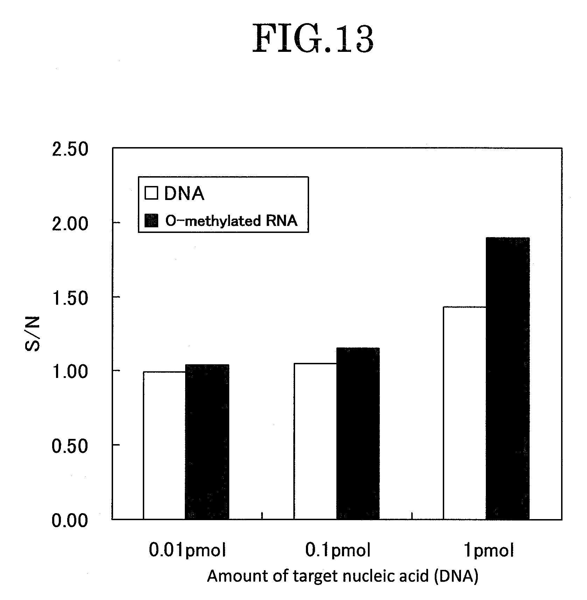

FIG. 13 is a diagram of signal-to-noise ratios (S/N) calculated in the measurement of the modified nucleobase of each of the concentrations using the nucleic acid probe. The used target nucleic acid and nucleic acid probe are similar to those in FIG. 12.

### EMBODIMENTS FOR CARRYING OUT THE INVENTION

The present invention provides a method for measuring a modified nucleobase. The method of the present invention includes:
(1) incubating a nucleic acid sample and a heterogeneous nucleic acid probe in a solution; and
(2) measuring the modified nucleobase using an antibody against the modified nucleobase in the solution obtained at (1).

The nucleic acid sample is a sample containing a target nucleic acid containing a modified nucleobase or a sample suspected to contain the target nucleic acid. The nucleic acid sample may also be an organism-derived biological sample, an environmental sample, or the like. Examples of the organism from which the biological sample is derived include animals such as mammals (e.g., humans, monkeys, mice, rats, rabbits, cattle, pigs, horses, goats, sheep) and birds (e.g., chickens), insects, microorganisms, plants, fungi, and fishes. The biological sample may also be a blood-related sample that is blood itself or a blood-derived sample (e.g., whole blood, blood serum, blood plasma), saliva, urine, milk, tissue or cell extract, or a combination thereof. The biological sample may further be derived from mammals contracting diseases (e.g., cancer, leukemia) or mammals that may contract diseases. Examples of the environmental sample include samples derived from soil, sea water, and fresh water that may contain nucleic acids. These samples may be subjected to another treatment before being used in the method of the present invention. Examples of the treatment include extraction and fragmentation (e.g., treatment with an enzyme such as a restriction enzyme) of nucleic acids (e.g., DNA such as genomic DNA, RNA). Consequently, the method of the present invention may further include extracting a nucleic acid from the nucleic acid sample and/or fragmenting the nucleic acid. The method of the present invention may also further include treating the sample by centrifugation, extraction, filtration, precipitation, heating, freezing, refrigeration, stirring, or the like.

The target nucleic acid is DNA or RNA, and DNA is preferable. The target nucleic acid is also a coding region or a non-coding region (e.g., a transcriptional regulation region) of DNA. The number of nucleotide residues composing the target nucleic acid (that is, the length of the target nucleic acid) is not limited to a particular number so long as it enables hybridization with the heterogeneous nucleic acid probe and may be 10 or more, preferably 15 or more, and more preferably 20 or more, for example. The number of nucleotides composing the target nucleic acid is also not limited to a particular number and may be any number that may occur by fragmentation of genomic DNA, for example. The number of the nucleotides composing the target nucleic acid may be 10,000 or less, 5,000 or less, 2,000 or less, 1,000 or less, 500 or less, 200 or less, or 100 or less, for example. A GC content of the target nucleic acid is not limited to a particular value and may be 10% or more, 20% or more, 30% or more, 40% or more, 50 % or more, or 60% or more, for example. The GC content of the target nucleic acid is also 90% or less, 80% or less, or 70% or less, for example. The number of the modified nucleic acid that the target nucleic acid contains is not limited to a particular number so long as it is one or more (e.g., 1 to 100, 1 to 20, 1 to 10, or 1 to 5).

In the present invention, the modified nucleic acid refers to a nucleobase having a structure in which a normal nucleobase selected from the group consisting of adenine (A), guanine (G), cytosine (C), thymine (T), and uracil (U) is modified. When the target nucleic acid is DNA, examples of the term "nucleobase" in the expression "modified nucleobase" include adenine (A), guanine (G), cytosine (C), and thymine (T). When the target nucleic acid is RNA, examples thereof include adenine (A), guanine (G), cytosine (C), and uracil (U). The nucleobase is preferably cytosine (C). Examples of modification include introduction of a substituent to the normal nucleobase, elimination of a group (e.g., an amino group, an oxo group, a methyl group) that the normal nucleobase has, and exchange of a group that the normal nucleobase has with a substituent. The substituent is not limited to a particular type so long as it is one that naturally occurring nucleobases can have, and examples thereof include the substituents that the modified nucleobases in the modified nucleotides described in Administrative Instructions under the Patent Cooperation Treaty (the version enforced on January 1, 2009), Annex C, Appendix 2, Table 2: List of Modified Nucleotides have. The modified nucleotides described in the literature can be the same as the modified nucleotides described in "Guidelines for Preparation of Specifications Containing Nucleotide Sequences or Amino Acid Sequences (July of 2002) or (December of 2009)," Annex 2, Table 2: Modified Base Table disclosed by the Japan Patent Office. Consequently, concerning the modified nucleobase, the guidelines can also be referred to. The substituent is preferably methyl, hydroxymethyl, or carboxy and more preferably methyl or hydroxymethyl. The position of the modification such as substitution is not limited to a particular position and is the 2-position or the 4- to 6-positions, for example, and preferably the 5-position for the nucleobase (C, T, or U) having a pyrimidine ring and is the 2-position, the 6-position, or the 8-position, for example, for the nucleobase (A or G) having a purine ring.

The modified nucleobase is not limited to a particular type so long as it can naturally occur, and examples thereof include the modified nucleobases that the modified nucleotides described in Administrative Instructions under the Patent Cooperation Treaty (the version enforced on January 1, 2009), Annex C, Appendix 2, Table 2: List of Modified Nucleotides have. The modified nucleotides described in the literature can be the same as the modified nucleotides described in the guidelines, Annex 2, Table 2: Modified Base Table. Consequently, concerning the modified nucleobase, the guidelines can also be referred to. The modified nucleobase is preferably methylcytosine (e.g., 5-methylcytosine), hydroxymethylcytosine (e.g., 5-hydroxymethylcytosine), or carboxylcytosine (e.g., 5-carboxylcytosine). The modified nucleobase is more preferably methylcytosine (e.g., 5-methylcytosine) or hydroxymethylcytosine (e.g., 5-hydroxymethylcytosine). It is known that the modified nucleobase brings changes in functions of nucleic acids (e.g., a change in the transcriptional regulation capability of a certain gene).

The heterogeneous nucleic acid probe is a probe that includes a nucleic acid heterogeneous with respect to the target nucleic acid and can detect the target nucleic acid through hybridization. The term "heterogeneous" means that the heterogeneous nucleic acid probe has a backbone structure different from a backbone structure (a structure composed of a sugar moiety and a phosphoric acid moiety) of the target nucleic acid as part or the whole of the backbone structure. Consequently, the type of the heterogeneous nucleic acid probe is determined in accordance with the type of the target nucleic acid. When the target nucleic acid is DNA, for example, a nucleic acid probe other than a DNA probe can be used as the heterogeneous nucleic acid probe. When the target nucleic acid is natural RNA, a nucleic acid probe other than a normal RNA probe composed of RNA homogeneous with the natural RNA can be used as the heterogeneous nucleic acid probe. Since DNA is preferable as the target nucleic acid, the heterogeneous nucleic acid probe is preferably a nucleic acid probe other than a DNA probe.

Examples of the heterogeneous nucleic acid probe include DNA probes, RNA probes, peptide nucleic acid (PNA) probes, locked nucleic acid (also called LNA or bridged nucleic acid (BNA)) probes, phosphorothioate (S-) nucleic acid probes, and chimera nucleic acid probes in which two or more such nucleic acid probes are coupled and/or mixed with each other (the chimera nucleic acid probe inevitably contains a nucleic acid heterogeneous with respect to the target nucleic acid). Examples of the RNA probes include a normal RNA probe composed of a natural ribonucleotide having a hydroxy group at the 2'-position and a modified RNA probe composed of a ribonucleotide the 2'-position hydroxy group of which is modified. The modified RNA probe may be a ribonuclease-resistant RNA probe. Examples of the modified RNA probe include a 2'-O-alkylated RNA probe. The 2'-O-alkylated RNA probe is preferably 2'-O-C₁₋₆ alkylated RNA probe. The C₁₋₆ alkyl group of the C₁₋₆ alkylation is a linear, branched, or cyclic C₁₋₆ alkyl group, and examples thereof include a methyl group, an ethyl group, a propyl group (e.g., n-propyl, iso-propyl), a butyl group (e.g., n-butyl, iso-butyl, sec-butyl, tert-butyl), a pentyl group, and a hexyl group. In terms of easiness of manufacture and acquisition or the like, the 2'-O-C-₆ alkylated RNA probe is a 2'-O-methylated RNA probe.

The number of nucleotide residues composing the heterogeneous nucleic acid probe (that is, the length of the heterogeneous nucleic acid probe) is not limited to a particular number so long as it is long enough for the hybridization with the target nucleic acid and may be 10 or more, preferably 15 or more, and more preferably 20 or more, for example. The number of nucleotides composing the heterogeneous nucleic acid probe may also be 100 or less, 80 or less, 60 or less, 50 or less, 40 or less, or 30 or less, for example. A GC content of the target nucleic acid is not limited to a particular value and may be 10% or more, 20% or more, 30% or more, 40% or more, 50% or more, or 60% or more, for example. The GC content of the target nucleic acid may also be 90% or less, 80% or less, or 70% or less, for example. The heterogeneous nucleic acid probe can be prepared by a method of synthesizing a probe known in the relevant field, for example.

The heterogeneous nucleic acid probe is used in the form of being free or the form of being immobilized to a solid phase (described below). Consequently, the heterogeneous nucleic acid probe may be labeled with a substance or group that enables immobilization to the solid phase. The labeling is performed either at the 5'-end or the 3'-end, for example. Examples of the substance or group that enables immobilization to the solid phase include substances or groups that enable covalent binding to the solid phase and affinity substances. Examples of the substances or groups that enable covalent binding to the solid phase include a thiol group or substances having a thiol group (the thiol group introduced into the heterogeneous nucleic acid probe can bind to a maleimide group on the solid phase) and an amino group or substances having an amino group (the amino group introduced into the heterogeneous nucleic acid probe can bind to maleic anhydride on the solid phase). Examples of the affinity substances include streptavidin, biotin, digoxigenin, dinitrophenol, fluorescein, and fluorescein isothiocyanate. In this case, a solid phase coated with another affinity substance having affinity with the affinity substance that the capture probe has can be used.

At the step (1), the incubation is performed in an appropriate solution on the condition that, when the target nucleic acid is contained in the nucleic acid sample, a hybridization reaction of the heterogeneous nucleic acid probe (being free or immobilized to the solid phase described below) and the target nucleic acid in the nucleic acid sample is made possible. As the solution, a buffer solution containing a salt (e.g., sodium citrate) and other components (e.g., a surfactant) can be used, for example. A hybridization temperature is 15°C to 95°C, for example (preferably 25°C to 65°C).

When the nucleic acid sample does not contain the target nucleic acid, even when the nucleic acid sample and the heterogeneous nucleic acid probe are incubated in the solution, a heterogeneous nucleic acid hybrid is not formed. In this case, the modified nucleobase cannot be detected at the step (2) described below, but it can be determined that the modified nucleobase is not present in the nucleic acid sample.

When the nucleic acid sample contains the target nucleic acid not containing the modified nucleobase (in other words, the target nucleic acid containing non-modified nucleobases alone), by incubating the nucleic acid sample and the heterogeneous nucleic acid probe in the solution, the target nucleic acid not containing the modified nucleobase and the heterogeneous nucleic acid probe react with each other, whereby a heterogeneous nucleic acid hybrid composed of the target nucleic acid and the heterogeneous nucleic acid probe is formed. In this case, the modified nucleobase cannot be detected at the step (2) described below, but it can be determined that the modified nucleobase is not present in the nucleic acid sample (even though the target nucleic acid is present) or, in other words, that a certain nucleobase in the target nucleic acid is not modified.

When the nucleic acid sample contains the target nucleic acid containing the modified nucleobase, by incubating the nucleic acid sample and the heterogeneous nucleic acid probe in the solution, the target nucleic acid containing the modified nucleobase and the heterogeneous nucleic acid probe react with each other, whereby a heterogeneous nucleic acid hybrid composed of the target nucleic acid and the heterogeneous nucleic acid probe is formed. In this case, it can be determined that the modified nucleobase is present at the step (2) described below, and the modified nucleobase can also be quantified.

In the present invention, the heterogeneous nucleic acid hybrid is a hybridization complex having a double-stranded structure of the target nucleic acid and the heterogeneous nucleic acid probe formed through hybridization. Examples of the structure of the heterogeneous nucleic acid hybrid are as illustrated in the following Table 1. The double-stranded structure may be formed at the entire region of the target nucleic acid and the heterogeneous nucleic acid probe as illustrated in (a). The double-stranded structure may also be formed at a partial region of the target nucleic acid or the heterogeneous nucleic acid probe as illustrated in (b) to (i). The heterogeneous nucleic acid hybrid may have a single-stranded structure of the target nucleic acid either at a 5'-end region or a 3'-end region [e.g., (b) to (e)] or may also have a single-stranded structure of the target nucleic acid both at the 5'-end region and the 3'-end region [e.g., (i)] in addition to the double-stranded structure, for example. The heterogeneous nucleic acid hybrid may also have a single-stranded structure of the heterogeneous nucleic acid probe either at the 5'-end region or the 3'-end region [e.g., (b), (c), (f), (g)] or may also have a single-stranded structure of the target nucleic acid both at the 5'-end region and the 3'-end region [e.g., (h)] in addition to the double-stranded structure. The single-stranded structure is a structure formed by either one or both of the target nucleic acid and the heterogeneous nucleic acid probe, which have formed the heterogeneous nucleic acid hybrid, having a non-hybridized region of one or more nucleotide residues at the 5'-end and/or the 3'-end.

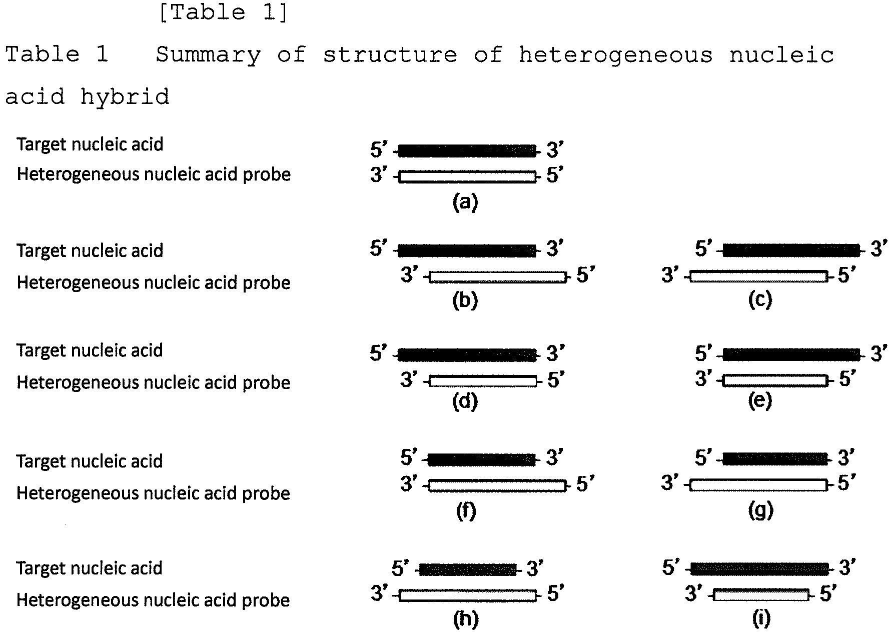

In the heterogeneous nucleic acid hybrid, the number of nucleotide residues of the target nucleic acid and the heterogeneous nucleic acid probe corresponding to the double-stranded structure part (that is, the length of the double-stranded structure part) is not limited to a particular number so long as it is long enough to enable hybridization with the target nucleic acid and may be 10 or more, preferably 15 or more, and more preferably 20 or more, for example. The number of the nucleotide residues of the target nucleic acid and the heterogeneous nucleic acid probe corresponding to the double-stranded structure part may also be 100 or less, 80 or less, 60 or less, or 50 or less, 40 or less, or 30 or less, for example. A GC content in the double-stranded structure part is not limited to a particular value and may be 10% or more, 20% or more, 30% or more, 40% or more, 50% or more, or 60% or more, for example. The GC content in the double-stranded structure part may also be 90% or less, 80% or less, or 70% or less, for example.

In the heterogeneous nucleic acid hybrid, the number of nucleotide residues of the target nucleic acid and the heterogeneous nucleic acid probe corresponding to the singe-stranded structure parts at its 5'-end region or 3'-end region (that is, the length of each of the single-stranded parts) is not limited to a particular number so long as it is one or more and is two or more, three or more, four or more, five or more, six or more, seven or more, eight or more, nine or more, 10 or more, 15 or more, 20 or more, or 50 or more, for example. The number is not limited to a particular number and may also be 10,000 or less, 5,000 or less, 2,000 or less, 1,000 or less, 500 or less, 200 or less, or 100 or less, for example. The heterogeneous nucleic acid probe may be designed such that the single-stranded structure part is formed at the 5'-end region or the 3'-end region in the heterogeneous nucleic acid hybrid.

In one embodiment, the heterogeneous nucleic acid probe may be designed such that an unpaired part of the modified nucleobase is formed in the double-stranded structure part of the heterogeneous nucleic acid hybrid. The unpaired part of the modified nucleobase can be introduced in order to facilitate detection of the modified nucleobase by an antibody. To form the unpaired part, a heterogeneous nucleic acid probe having a nucleotide sequence that is not perfectly complementary with respect to the target nucleic acid in the double-stranded structure part may be used, for example.

An example of the heterogeneous nucleic acid probe in which the unpaired part of the modified nucleobase is formed in the double-stranded structure part of the heterogeneous nucleic acid hybrid is a heterogeneous nucleic acid probe lacking a nucleotide residue complementary with respect to a nucleotide residue having a modified nucleobase in the target nucleic acid [e.g., (I) in Table 2]. The heterogeneous nucleic acid probe may be a heterogeneous nucleic acid probe lacking one nucleotide residue alone complementary with respect to the nucleotide residue having the modified nucleobase in the target nucleic acid [e.g., (I-1) in Table 2] or a heterogeneous nucleic acid probe lacking two or more (2 to 20, 2 to 10, or 2 to 5, for example) adjacent nucleotide residues containing the nucleotide residue having the modified nucleobase in the target nucleic acid [e.g., (I-2) in Table 2]. The number of the nucleotide residue having the modified nucleobase in the unpaired part is not limited to a particular number so long as it is one or more as described above. Concerning the nucleic acid probe, refer to Patent Literature 1 and Non Patent Literature 2, for example. When the position of the nucleotide residue having the modified nucleobase in the target nucleic acid to be measured is determined, such design is made possible.

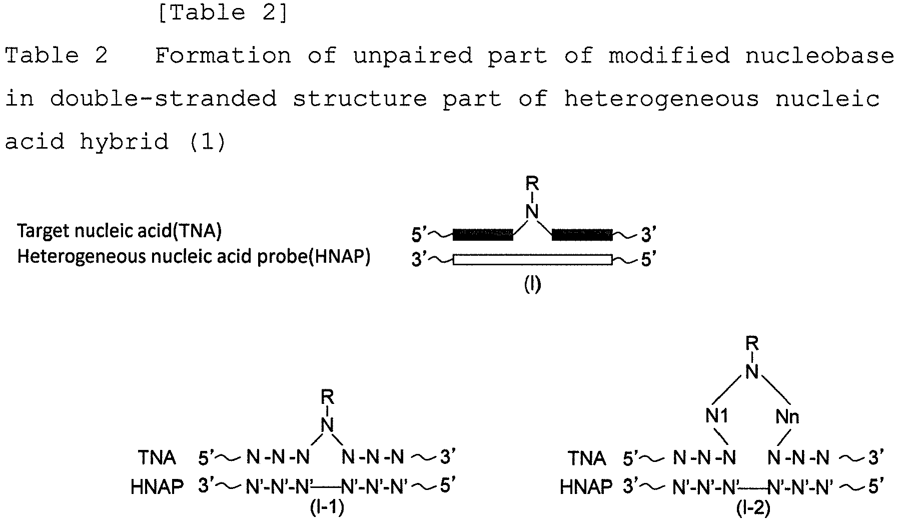

R-N: Nucleotide residue having modified nucleobase
N: Nucleotide residue having non-modified nucleobase composing target nucleic acid
N': Nucleotide residue composing heterogeneous nucleic acid probe
n in Nn: Number of nucleotide residues in bulge (loop) part (e.g., 2 to 20. When n is 2, either N1 or N2 or both N1 and N2 may have substituent R.)
R: Substituent that nucleobase has

Another example of the heterogeneous nucleic acid probe in which the unpaired part of the modified nucleobase is formed in the double-stranded structure part of the heterogeneous nucleic acid hybrid is a heterogeneous nucleic acid probe having a nucleotide residue noncomplementary with respect to the nucleotide residue having the modified nucleobase in the target nucleic acid [e.g., (I') in Table 2]. The heterogeneous nucleic acid probe may be a heterogeneous nucleic acid probe having one nucleotide residue alone noncomplementary with respect to the nucleotide residue having the modified nucleobase in the target nucleic acid [e.g., (I'-1) in Table 2] or a heterogeneous nucleic acid probe in which two or more (2 to 20, 2 to 10, or 2 to 5, for example) adjacent nucleotide residues including the nucleotide residue having the modified nucleobase in the target nucleic acid are noncomplementary [e.g., (I'-2) in Table 2]. When the position of the nucleotide residue having the modified nucleobase in the target nucleic acid to be measured is determined, such design is made possible.

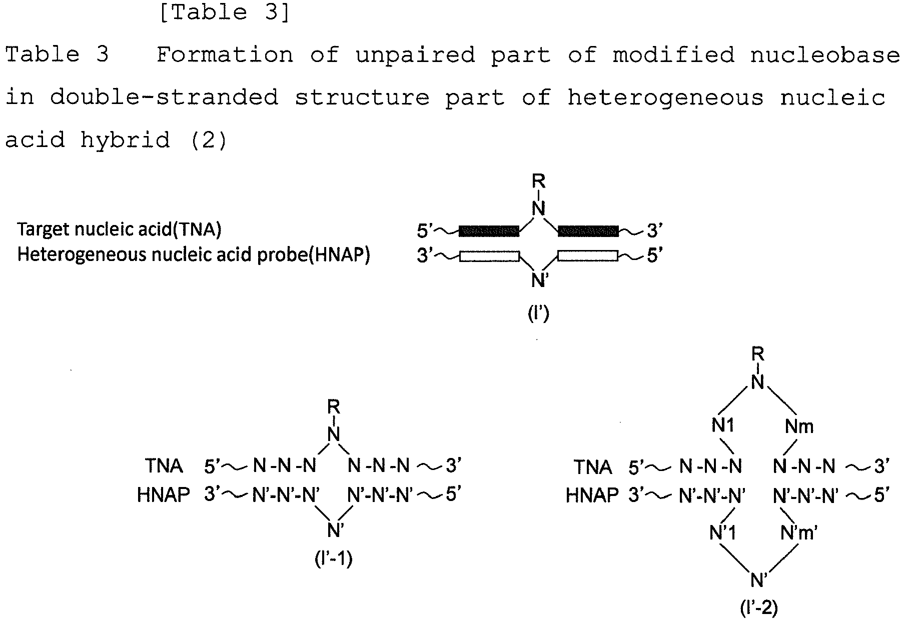

R-N: Nucleotide residue having modified nucleobase
N: Nucleotide residue having non-modified nucleobase composing target nucleic acid
N': Nucleotide residue composing heterogeneous nucleic acid probe
m in Nm: Number of nucleotide residues in non-complementary part (N1 to Nm) (e.g., 2 to 20. When m is 2, either N1 or N2 or both N1 and N2 may have substituent R.)
m' in N'm': Number of nucleotide residues in non-complementary part (N'1 to N'm') (e.g., 2 to 20)
R: Substituent that nucleobase has

In another embodiment, the heterogeneous nucleic acid probe may be designed such that the modified nucleobase is present in the single-stranded structure part of the heterogeneous nucleic acid hybrid. In the single-stranded structure part (the target nucleic acid) of the heterogeneous nucleic acid hybrid, the modified nucleobase may be present at a non-end part of the single-stranded structure part on the 5'-end side [e.g., (II) in Table 4], may be present at an end part of the single-stranded structure part on the 5'-end side [e.g., (III) in Table 4], may be present at a non-end part of the single-stranded structure part on the 3'-end side [e.g., (IV) in Table 4], may be present at an end part of the single-stranded structure part on the 3'-end side [e.g., (V) in Table 4], or may be any combination of two, three, or four of these. The heterogeneous nucleic acid probe may be designed so that the single-stranded structure part (the target nucleic acid) of the heterogeneous nucleic acid hybrid will contain the modified nucleobase in such a manner. The number of the nucleotide residue having the modified nucleobase in the single-stranded structure part is not limited to a particular number so long as it is one or more as described above. Alternatively, the heterogeneous nucleic acid probe may be designed so that the modified nucleobase will be present in the single-stranded structure part of the heterogeneous nucleic acid hybrid and that the unpaired part of the modified nucleobase will further be formed in the double-stranded structure part of the heterogeneous nucleic acid hybrid as described above. When the position of the nucleotide residue having the modified nucleobase in the target nucleic acid to be measured is determined, such design is made possible.

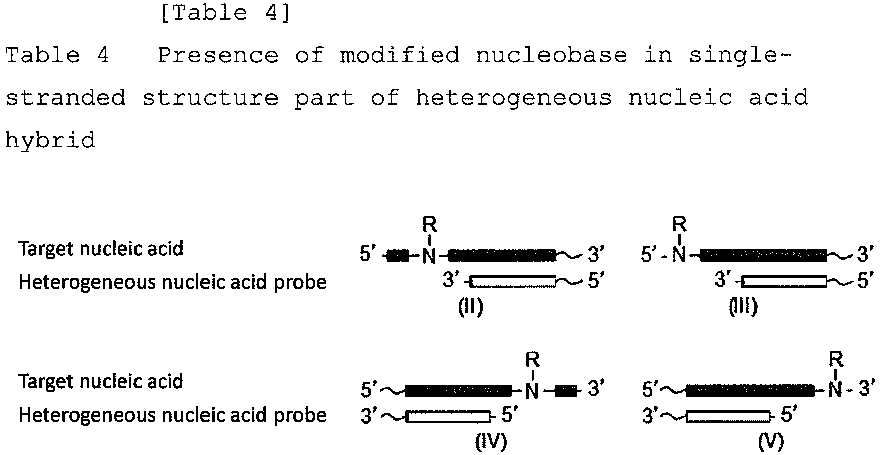

R-N: Nucleotide residue having modified nucleobase N: Nucleotide residue having non-modified nucleobase
R: Substituent that nucleobase has

The method of the present invention may further include adding the heterogeneous nucleic acid probe to a solution containing the nucleic acid sample to prepare a solution containing both the nucleic acid sample and the heterogeneous nucleic acid probe. The heterogeneous nucleic acid probe can be added to the nucleic acid sample in the form of a solid or as a solution.

When the solution for incubation is prepared from the nucleic acid sample containing the target nucleic acid containing the modified nucleobase, the concentration of the target nucleic acid in the solution is not limited to a particular value so long as it is detectable by the method of the present invention and may be 0.01 nM or more, preferably 0.1 nM or more, more preferably 1 nM or more, further more preferably 5 nM or more, and particularly preferably 10 nM or more, for example. The concentration of the target nucleic acid in the solution may also be 1 M or less, 100 mM or less, 10 mM or less, 1 mM or less, 100 µM or less, 10 µM or less, or 1 µM or less, for example. Since it has been revealed that the method of the present invention provides an especially excellent effect when the concentration of the target nucleic acid in the solution is 1 nM or more in the method of the present invention (refer to Example 1), it is also preferable that the concentration of the target nucleic acid be 1 nM or more, for example. Since the concentration of the target nucleic acid in the solution is unknown in many cases, it may be difficult to strictly set a concentration of the target nucleic acid. Depending on the type of the nucleic acid sample, the concentration of the target nucleic acid that can be contained in the nucleic acid sample can empirically be predicted to some extent, or the concentration of the target nucleic acid is determined (in a case when although the size and/or concentration of the target nucleic acid is separately measured, the presence or absence of the modified nucleobase in the target nucleic acid and the content of the modified nucleobase in the target nucleic acid are unknown, for example). In such cases, setting of the concentration of the target nucleic acid may be attempted as described above.

The concentration of the heterogeneous nucleic acid probe in the solution is not limited to a particular value so long as the target nucleic acid is detectable by the method of the present invention and may be 0.01 nM or more, preferably 0.1 nM or more, more preferably 1 nM or more, further more preferably 5 nM or more, and particularly preferably 10 nM or more, for example. The concentration of the heterogeneous nucleic acid probe in the solution may also be 1 M or less, 100 mM or less, 10 mM or less, 1 mM or less, 100 µM or less, 10 µM or less, or 1 µM or less, for example. Consequently, the heterogeneous nucleic acid probe may be added to the solution so that such a concentration will be achieved.

In a preferable embodiment, the target nucleic acid may be a target nucleic acid potentially containing two or more modified nucleobases. The number of the modified nucleobases potentially contained in the target nucleic acid is not limited to a particular number so long as it is two or more and is 2 to 30, 2 to 20, 2 to 10, or 2 to 5 (e.g., 2, 3, 4, or 5), for example. When a plurality of modified nucleobases are contained in the target nucleic acid, it has been revealed that even when the concentration of the target nucleic acid in the solution used for the hybridization of the target nucleic acid and a heterogeneous nucleic acid probe is extremely low (e.g., 0.1 nM or more), the modified nucleobases can be measured with high sensitivity. Consequently, the method of the present invention can use a heterogeneous nucleic acid probe that is designed so as to hybridize with the target nucleic acid potentially containing two or more modified nucleobases. When the number of nucleobases potentially modified in the target nucleic acid to be measured is determined, such design is made possible.

The modified nucleobase is measured using an antibody against the modified nucleobase in a solution containing the heterogeneous nucleic acid hybrid. In the measurement, although the solution obtained at the step (1) may be used as it is, addition of another solution and/or replacement of the solution with another solution may be performed in order to perform measurement in a solution more suitable for the measurement of the modified nucleobase by the antibody. The replacement can be performed by adding the solution obtained at the step (1) to a solid phase, immobilizing the heterogeneous nucleic acid hybrid that can be contained in the solution to the solid phase, removing the solution from the solid phase, washing the solid phase with a cleaning liquid as needed, and adding another solution (e.g., a solution containing the antibody against the modified nucleobase) thereto, for example. The solution used in the measurement is not limited to a particular type so long as it is a solution suitable for an antigen-antibody reaction.

The measurement can be performed by immunological methodology. Examples of the immunological methodology include an enzyme immunoassay (EIA) (e.g., direct competitive ELISA, indirect competitive ELISA, and sandwich ELISA), a radioimmunoassay (RIA), a fluoroimmunoassay (FIA), immunochromatography, a luminescence immunoassay, a spin immunoassay, Western blot, and latex agglutination.

The antibody against the modified nucleobase may be a polyclonal antibody or a monoclonal antibody. The antibody against the modified nucleobase may be any isotype of immunoglobulin (e.g., IgG, IgM, IgA, IgD, IgE, IgY). The antibody against the modified nucleobase may be a full-length antibody. The full-length antibody refers to an antibody containing a heavy chain and a light chain, each of the chains containing a variable region and a constant region (e.g., an antibody containing two Fab parts and an Fc part). The antibody against the modified nucleobase may also be an antibody fragment derived from the full-length antibody. The antibody fragment is part of the full-length antibody, and examples thereof include F(ab')₂, Fab', Fab, and Fv. The antibody against the modified nucleobase may also be a modified antibody such as a single-stranded antibody. The antibody against the modified nucleobase may further be an antibody used as a primary antibody in an immunoassay such as ELISA, and in this case, a secondary antibody is used in combination.

The antibody against the modified nucleobase may have affinity for a nucleoside having the modified nucleobase (a structural unit composed of the modified nucleobase and 2'-deoxyribose or ribose), a nucleotide having the modified nucleobase (a structural unit composed of the modified nucleobase, 2'-deoxyribose or ribose, and phosphate), or two or more nucleotides containing the nucleotide having the modified nucleobase (e.g., an oligonucleotide composed of two to five nucleotides). Examples of the antibody against the modified nucleobase when the target nucleic acid is DNA include 1) antibodies against a deoxyribonucleoside having a modified nucleobase selected from the group consisting of 2'-deoxy-modified adenosine, 2'-deoxy-modified guanosine, 2'-deoxy-modified cytidine, and 2'-deoxy-modified thymidine, 2) antibodies against a deoxyribonucleotide having a modified nucleobase selected from the group consisting of 2'-deoxy-modified adenosine 5'-phosphate, 2'-deoxy-modified guanosine 5'-phosphate, 2'-deoxy-modified cytidine 5'-phosphate, and 2'-deoxy-modified thymidine 5'-phosphate, and 3) antibodies against two or more deoxyribonucleotides containing the above deoxyribonucleotide having the modified nucleobase. Examples of the antibody against the modified nucleobase when the target nucleic acid is RNA include 1') antibodies against a nucleoside having a modified nucleobase selected from the group consisting of modified adenosine, modified guanosine, modified cytidine, modified thymidine, and modified uridine, 2') antibodies against a ribonucleotide having a modified nucleobase selected from the group consisting of modified adenosine 5'-phosphate, modified guanosine 5'-phosphate, modified cytidine 5'-phosphate, and modified uridine 5'-phosphate and 3') antibodies against two or more ribonucleotides containing the above ribonucleotide having the modified nucleobase.

For the antibody against the modified nucleobase, an antibody that is prepared by using a complex of the nucleoside having the modified nucleobase, the nucleotide having the modified nucleobase, or the two or more nucleotides containing the nucleotide having the modified nucleobase and a carrier protein (e.g., BSA, KLH) as an antigen and recognizes at least part of backbone structures of nucleic acids can be used, for example. Since various antibodies against the modified nucleobase prepared using such complexes are commercially available, the method of the present invention may use a commercially available antibody, for example. The method of the present invention may also use the antibody against the modified nucleobase prepared as follows, for example.

The polyclonal antibody against the modified nucleobase can be acquired by administering the above complex as the antigen together with a commercially available adjuvant (e.g., a complete or incomplete Freund's adjuvant) to an animal subcutaneously or intra-abdominally about two to four times every 2 to 3 weeks, collecting whole blood about 3 to about 10 days after the final immunity, and purifying an antiserum, for example. Examples of the animal to which the antigen is administered include mammals such as rats, mice, rabbits, goats, cattle, guinea pigs, and hamsters.

The monoclonal antibody against the modified nucleobase can be prepared by cell fusion, for example.
The above complex is administered together with a commercially available adjuvant to a mouse subcutaneously or intra-abdominally two to four times, collecting the spleen or a lymph node about three days after the final administration, and collecting white blood cells, for example. These white blood cells and a myeloma cell (e.g., NS-1) are subjected to cell fusion to obtain a hybridoma producing a monoclonal antibody against the factor. Examples of the cell fusion include a PEG method and a voltage pulse method. The hybridoma producing a desired monoclonal antibody can be selected by detecting an antibody that specifically binds to an antigen using known EIA, RIA, or the like in cultivated supernatant. Cultivation of the hybridoma producing the monoclonal antibody can be performed in vitro or in vivo such as in a mouse, a rat, or preferably mouse ascites, and the antibody can be acquired from the cultivated supernatant of the hybridoma or animal ascites. The monoclonal antibody may be any isotype of IgG, IgM, IgA, IgE, and the like. Alternatively, in vitro methods such as a phage display method (Ulman et al, Proc. Natl. Acad. Sci. U.S.A., 90, 1184-89 (1993)) and an ADLib system (WO2004/011644) are also known as methods for preparing a monoclonal antibody, and such methods may be used to prepare the antibody against the modified nucleobase.

The antibody against the modified nucleobase may be used while being immobilized to a solid phase. Examples of the solid phase include supports such as particles (e.g., magnetic particles), membranes (e.g., a nitrocellulose membrane), glass, plastic, and metal, containers such as plates (e.g., a multiwell plate), and devices. The antibody may also be provided in the form of being impregnated into a medium such as filter paper. The antibody against the modified nucleobase may be labeled with a labeling substance. Examples of the labeling substance include enzymes (e.g., peroxidase, alkaline phosphatase, luciferase, β-galactosidase), affinity substances (e.g., streptavidin and biotin), fluorescent substances or proteins (e.g., fluorescein, fluorescein isothiocyanate, rhodamine, green fluorescent protein, red fluorescent protein), luminescent substances (e.g., luciferin, aequorin), and radioactive substances (e.g., ³H, ¹⁴C, ³²P, ³⁵S, ¹²⁵I). When a secondary antibody is used in the method of the present invention, the secondary antibody may be labeled with such a labeling substance.

The measurement of the modified nucleobase by the antibody against the modified nucleobase is performed qualitatively or quantitatively, and the presence or absence or the amount of the modified nucleobase can be evaluated. In the present invention, the measurement of the modified nucleobase intends not only the measurement of the modified nucleobase itself but also the measurement of the target nucleic acid containing the modified nucleobase.

The measurement of the presence or absence of the modified nucleobase may be perfumed as follows, for example:
(2-1) in the solution obtained at the step (1), performing an assay using the antibody against the modified nucleobase to measure a signal value;
(2-2) in a solution that does not contain the target nucleic acid containing the modified nucleobase and contains the heterogeneous nucleic acid probe, performing an assay using the antibody against the modified nucleobase to measure a background value; and
(2-3) comparing the signal value with the background value to evaluate the presence or absence of the modified nucleobase.

In the measurement of the modified nucleobase, the signal value and the background value are values (e.g., absorbance, the degree of fluorescence, the degree of coloration, radioactivity) that are measured using a label binding to the antibody against the modified nucleobase or the secondary antibody (when the secondary antibody is used).

The measurement of the amount of the modified nucleobase may be performed together with the measurement of the background value, for example. Specifically, the measurement of the amount of the modified nucleobase may be performed as follows:
(2-1') in the solution obtained at the step (1), performing an assay using the antibody against the modified nucleobase to measure a signal value;
(2-2') in a solution that does not contain the target nucleic acid containing the modified nucleobase and contains the heterogeneous nucleic acid probe, performing an assay using the antibody against the modified nucleobase to measure a background value;
(2-3') correcting the signal value with the background value to obtain a corrected signal value; and
(2-4') based on the corrected signal value, evaluating the amount of the modified nucleobase.

Alternatively, the measurement of the amount of the modified nucleobase may be performed using a preparation. Specifically, the measurement of the amount of the modified nucleobase may be performed as follows:
(2-1") in the solution obtained at the step (1), performing an assay using the antibody against the modified nucleobase to measure a signal value;
(2-2") in a solution containing the target nucleic acid containing the modified nucleobase (preparation) and the heterogeneous nucleic acid probe, performing an assay using the antibody against the modified nucleobase to measure a value for calibration; and
(2-3") comparing the signal value with the value for calibration to evaluate the amount of the modified nucleobase.

The above measurement using the preparation may be performed in combination with the above measurement of the background value.

In a specific embodiment, the method of the present invention may be performed by ELISA. When the nucleic acid sample contains the target nucleic acid containing the modified nucleobase, for example, the method of the present invention by ELISA may be performed as follows:
(i) incubating the nucleic acid sample containing the target nucleic acid containing the modified nucleobase and a heterogeneous nucleic acid probe labeled with a first affinity substance in a solution to form a heterogeneous nucleic acid hybrid composed of the target nucleic acid and the heterogeneous nucleic acid probe;
(ii) immobilizing the heterogeneous nucleic acid hybrid to a solid phase treated with a second affinity substance;
(iii) reacting a primary antibody against the modified nucleobase with the heterogeneous nucleic acid hybrid immobilized to the solid phase to obtain a primary complex of the primary antibody and the heterogeneous nucleic acid hybrid;
(iv) reacting a secondary antibody labeled with a labeling substance with the primary complex to obtain a secondary complex of the secondary antibody and the primary antibody; and
(v) using the labeling substance that the secondary antibody in the secondary complex has, measuring the presence and/or the amount of the formed heterogeneous nucleic acid hybrid (in other words, the modified nucleobase).

The first affinity substance and the second affinity substance are used in a combination having mutual affinity (e.g., a combination of biotin and streptavidin). The method of the present invention may include (i') incubating the nucleic acid sample containing the target nucleic acid containing the modified nucleobase and the heterogeneous nucleic acid probe immobilized to a solid phase in a solution to form a heterogeneous nucleic acid hybrid composed of the target nucleic acid and the heterogeneous nucleic acid probe in place of the steps (i) and (ii). In this case, obtaining the heterogeneous nucleic acid probe immobilized to the solid phase (e.g., adding the heterogeneous nucleic acid probe labeled with the first affinity substance to the solid phase treated with the second affinity substance) may further be included. The method of the present invention may also include washing the solid phase before the step (iii). The secondary antibody may be an antibody that recognizes the primary antibody alone (e.g., an antibody that binds to the constant region of the primary antibody) and may also be an antibody that recognizes both the primary antibody in the secondary complex and the primary complex. The method of the present invention including (i) to (v) can be performed in accordance with the methodology described in detail in the specification.

The present invention also provides a kit for measuring a modified nucleobase. The kit of the present invention includes:
(I) a heterogeneous nucleic acid probe; and
(II) an antibody against the modified nucleobase.

The heterogeneous nucleic acid probe and the modified nucleobase are as described above. The heterogeneous nucleic acid probe may be labeled with an affinity substance, and the antibody against the modified nucleobase may be labeled with a labeling substance, for example. The kit of the present invention may further contain the components as described above including the affinity substance, the labeling substance, the secondary antibody, a detection reagent for the secondary antibody (e.g., when the secondary antibody is labeled with an enzyme, a substrate for the enzyme), and the solid phase. The solid phase may be treated with the affinity substance. The kit of the present invention may also contain the preparation of the modified nucleobase or the preparation of the target nucleic acid containing the modified nucleobase as solution or as powder.

The kit of the present invention contains the components in the form of being isolated from each other or in the form of being mixed with each other. In the kit of the present invention, the components may be provided in the form of being contained in different containers (e.g., a tube, a plate), for example. Alternatively, the kit of the present invention may be provided in the form of a device. Specifically, all the components may be provided in the form of being contained in a device. Alternatively, part of the components may be provided in the form of being contained in a device, whereas the rest may be provided in the form of not being contained in the device (e.g., the form of being contained in a different container). In this case, the component(s) not contained in the device may be used by being injected into the device in the measurement of a target substance.

### EXAMPLES

Although the following describes the present invention in more detail with reference to examples, the present invention is not limited to these examples.

### Example 1: Use of Heterogeneous Nucleic Acid Probe in Measurement of Modified Nucleobase (1)

The nucleotide sequence of the target nucleic acid (DNA) is 5'-TTGCGCGGCGTC[C]GTCCTGTTGACTTC-3' (SEQ ID NO: 1, [C] indicates 5-methylcytosine), and the nucleotide sequence of the heterogeneous nucleic acid probe for capturing the target nucleic acid is 5'-GAAGUCAACAGGACGACGCCGCGCAA-3' (SEQ ID NO:2, the backbone of the nucleic acid is normal RNA or 2'-O-methylated RNA, the 5'-end is biotin-labeled); those artificially synthesized by Hokkaido System Science Co., Ltd. were used. The heterogeneous nucleic acid probe was designed such that, when a heterogeneous nucleic acid hybrid with the target nucleic acid was formed, an unpaired part was formed at the modified nucleobase [C] in the double-stranded structure part of the heterogeneous nucleic acid hybrid.

The measurement of the modified nucleobase using the heterogeneous nucleic acid probe was carried out as follows. First, the target nucleic acid containing 5-methylcytosine (1 pmol, 0.1 pmol, or 0.01 pmol) and the heterogeneous nucleic acid probe (5 pmol) were dissolved in 100 µL of a hybridization buffer (4x SSC, 0.3% Tween20) (in a nucleic acid sample solution, the concentration of the target nucleic acid was 10 nM, 1 nM, or 0.1 nM, and the concentration of the heterogeneous nucleic acid probe was 50 nM) and were reacted at 60°C for 2 hours to form a heterogeneous nucleic acid hybrid composed of the target nucleic acid and the heterogeneous nucleic acid probe. A solution not containing the target nucleic acid was also prepared, and a similar operation was performed. The solution after the hybridization reaction in an amount of 100 µL was added to a streptavidin-coated plate (manufactured by Thermo Scientific) and was reacted at 37°C for 30 minutes to immobilize the heterogeneous nucleic acid hybrid on the streptavidin plate. The streptavidin plate was washed twice with 300 µL of PBS-T, and 50 ng/mL of an anti-methylcytosine antibody ((Clone33D3 manufactured by Nippon Gene Co., Ltd.). This antibody is an antibody that recognizes not only 5-methylcytosine but also at least part of a backbone structure of DNA (e.g., a structure composed of a repeating unit of a deoxyribose moiety and a phosphoric acid moiety)) was added thereto by 100 µL each and was reacted at 37°C for 1 hour. The streptavidin plate was washed three times with 300 µL of PBS-T, and 250 ng/mL of a peroxidase-labeled anti-IgG antibody (manufactured by Thermo Scientific) was added thereto by 100 µL each and was reacted at 37°C for 30 minutes. After the streptavidin plate was washed three times with 300 µL of PBS-T, 3,3',5,5'-tetramethylbenzidine was added thereto by 100 µL each and was reacted in a dark place at room temperature for 15 minutes. Thereafter, a 2N hydrochloric acid solution was added thereto by 100 µL each, and absorbance at 450 nm was measured by a microplate reader (Arvo manufactured by PerkinElmer, Inc.).

The target nucleic acid was measured by a similar method except that the homogeneous nucleic acid probe (SEQ ID NO:3: 5'-GAAGTCAACAGGACGACGCCGCGCAA-3') with DNA as the backbone was used in place of the heterogeneous nucleic acid probe with normal RNA or 2'-O-methylated RNA as the backbone.

As a result of the measurement, the heterogeneous nucleic acid probe reduced the background value of the detection signal compared with the homogeneous nucleic acid probe (Table 4 and FIG. 2) and increased the S/N value in the target nucleic acid of each concentration (Table 5 and FIG. 3). These facts indicate that use of the nucleic acid probe heterogeneous with respect to the target nucleic acid in combination is effective to increase detection sensitivity in the measurement of the target nucleic acid containing the modified nucleobase by the antibody. When the amount of the target nucleic acid was 0.1 pmol or more (that is, when the concentration of the target nucleic acid in the solution was set to 1 nM or more to cause hybridization with the heterogeneous nucleic acid probe and to form the heterogeneous nucleic acid hybrid) in particular, an especially excellent effect of the heterogeneous nucleic acid probe over the homogeneous nucleic acid probe was revealed (Table 5 and FIG. 3).

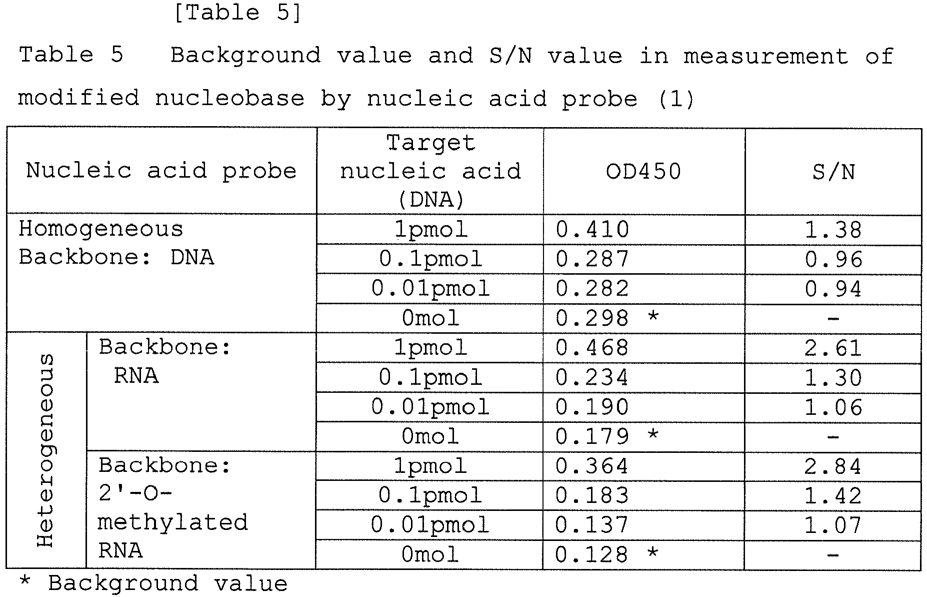

From the foregoing, it has been revealed that when the target nucleic acid containing the modified nucleobase is measured by the antibody, use of the heterogeneous nucleic acid probe in combination is effective.

### Example 2: Investigation of Difference in Antibody Clone

Tests were carried out by a method similar to that in Example 1 except that 100 ng/mL of an anti-methylcytosine antibody (Clone33D3 manufactured by Millipore Corporation), 100 ng/mL of an anti-methylcytosine antibody (Clone162 33 D3 manufactured by Millipore Corporation), and 10 ng/mL of an anti-methylcytosine antibody (Clone10G4 manufactured by Zymo Research Corporation) were used in place of the anti-methylcytosine antibody (Clone33D3 manufactured by Nippon Gene Co., Ltd.) used in Example 1. The reason why different concentrations were employed for the various antibodies is that the various antibodies varied in activity, detection signals substantially varied, and adjustment was performed so that comparable detection signals would be obtained.

As a result of the tests, reductions in the background value and increases in the S/N value were revealed for all the antibodies (Table 6 and FIGs. 4 and 5).

[Table 6]

**Table 6 Background value and S/N value in measurement of modified nucleobase by using various kinds of antibodies and nucleic acid probe in combination**

| Antibody | Nucleic acid probe | Target nucleic acid (DNA) | OD450 | S/N | Antibody concentration |
|---|---|---|---|---|---|
| 33D3 (Manufactured by Nippon Gene) | DNA | 1pmol | 0.698 | 1.33 | 75ng/mL |
| | | 0mol | 0.526 * | - | |
| | 2'-O-methylated RNA | 1pmol | 0.636 | 2.19 | |
| | | 0mol | 0.291 * | - | |
| 33D3 (Manufactured by Millipore) | DNA | 1pmol | 0.417 | 2.23 | 100ng/mL |
| | | 0mol | 0.187 * | - | |
| | 2'-O-methylated RNA | 1pmol | 0.279 | 2.53 | |
| | | 0mol | 0.110 * | | |
| 162 33 D3 | DNA | 1pmol | 0.526 | 2.37 | 100ng/mL |
| | | 0mol | 0.221 * | - | |
| | 2'-O-methylated RNA | 1pmol | 0.379 | 3.10 | |
| | | 0mol | 0.122 * | - | |
| 10G4 | DNA | 1pmol | 0.692 | 1.35 | 10ng/mL |
| | | Omol | 0.514 * | - | |
| | 2'-O-methylated RNA | 1pmol | 0.631 | 1.90 | |
| | | 0mol | 0.333 * | - | |

| | | | | | |
|---|---|---|---|---|---|
| * Background value | | | | | |

From the foregoing, it has been revealed that the effect of the present invention is not limited to a specific antibody clone.

### Example 3: Use of Heterogeneous Nucleic Acid Probe in Measurement of Modified Nucleobase (2)

The nucleotide sequence of the target nucleic acid (DNA) is 5'-AATCAG[C]GGGAGCTCTTTCTTTGCGCGGCGTCCGTCCTGTTGACTTC-3' (SEQ ID NO:4, [C] indicates 5-methylcytosine), and the nucleotide sequence of the heterogeneous nucleic acid probe for capturing the target nucleic acid is 5'-GAAGUCAACAGGACGACGCCGCGCAA-3' (SEQ ID NO:2, the backbone of the nucleic acid is normal RNA or 2'-O-methylated RNA, the 5'-end is biotin-labeled); those artificially synthesized by Hokkaido System Science Co., Ltd. were used. The heterogeneous nucleic acid probe was designed such that, when a heterogeneous nucleic acid hybrid with the target nucleic acid was formed, the modified nucleobase [C] was present in the single-stranded structure part of the heterogeneous nucleic acid hybrid.

The measurement of the modified nucleobase using the heterogeneous nucleic acid probe was carried out by a method similar to that in Example 1 except that a different target nucleic acid was used as described above.

The target nucleic acid was measured by a similar method except that the homogeneous nucleic acid probe (SEQ ID NO:3: 5'-GAAGTCAACAGGACGACGCCGCGCAA-3') with DNA as the backbone in place of the heterogeneous nucleic acid probe with normal RNA or 2'-O-methylated RNA as the backbone.

As a result of the measurement, when the heterogeneous nucleic acid hybrid with the target nucleic acid was formed, even when the heterogeneous nucleic acid probe was designed such that the modified nucleobase [C] was present in the single-stranded structure part of the heterogeneous nucleic acid hybrid, reductions in the background value and increases in the S/N value were revealed (Table 7 and FIGs. 6 and 7). When the amount of the target nucleic acid was 0.1 pmol or more (that is, when the concentration of the target nucleic acid in the solution was set to 1 nM or more to cause hybridization with the heterogeneous nucleic acid probe and to form the heterogeneous nucleic acid hybrid), an especially excellent effect of the heterogeneous nucleic acid probe over the homogeneous nucleic acid probe was revealed (Table 7 and FIG. 7).

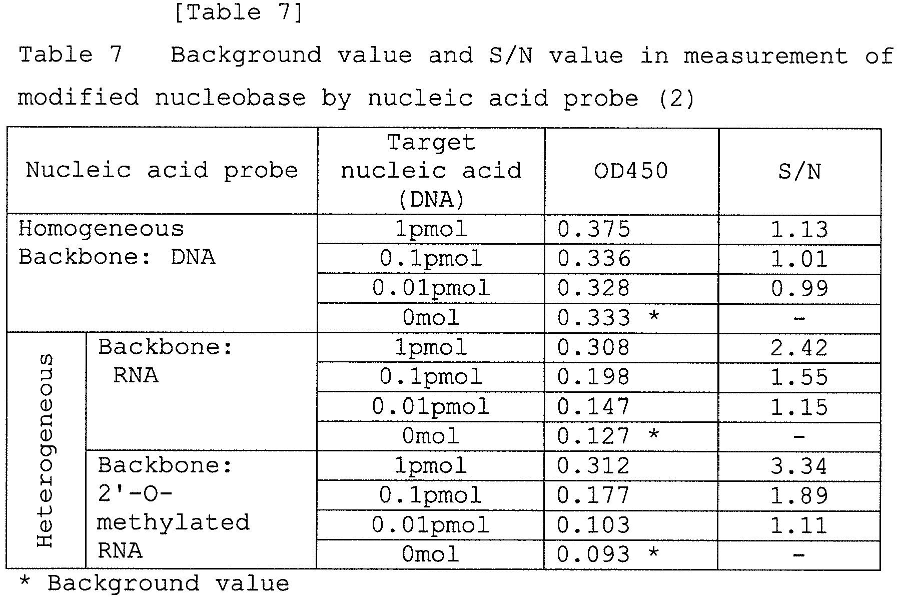

From the foregoing, it has been revealed that when the target nucleic acid containing the modified nucleobase is measured by the antibody, not only the heterogeneous nucleic acid probe (Example 1) that is designed such that the unpaired part is formed at the modified nucleobase [C] in the double-stranded structure part of the heterogeneous nucleic acid hybrid, but also the heterogeneous nucleic acid probe (Example 3) that is designed such that the modified nucleobase [C] is present in the single-stranded part of the heterogeneous nucleic acid hybrid is effective.

### Example 4: Use of Heterogeneous Nucleic Acid Probe in Measurement of Modified Nucleobase (3)

The nucleotide sequence of the target nucleic acid (DNA) is 5'-G[C]GGAGCTCTCCCT[C]GGGA[C]GGTGGCAGCCTCGAGTGGTCCTGCA-3' (SEQ ID NO:5, [C] indicates 5-methylcytosine), and the nucleotide sequence of the heterogeneous nucleic acid probe for capturing the target nucleic acid is 5'-UGCAGGACCACUCGAGGCUGCCAC-3' (SEQ ID NO:6, the backbone of the nucleic acid is 2'-O-methylated RNA, the 5'-end is biotin-labeled); those artificially synthesized by Hokkaido System Science Co., Ltd. were used. The heterogeneous nucleic acid probe was designed such that, when a heterogeneous nucleic acid hybrid with the target nucleic acid was formed, the modified nucleobase [C] was present in the single-stranded structure part of the heterogeneous nucleic acid hybrid.

The measurement of the modified nucleobase using the heterogeneous nucleic acid probe was carried out by a method similar to that in Example 1 except that a different target nucleic acid and a different heterogeneous nucleic acid probe were used as described above.

The target nucleic acid was measured by a similar method except that the homogeneous nucleic acid probe (SEQ ID NO:7: 5'-TGCAGGACCACTCGAGGCTGCCAC-3') with DNA as the backbone was used in place of the heterogeneous nucleic acid probe with 2'-O-methylated RNA as the backbone.

As a result of the measurement, even when the target nucleic acid and the heterogeneous nucleic acid probe different from those of Example 1 and Example 3 were used, reductions in the background value and increases in the S/N value were revealed (Table 8 and FIGs. 8 and 9). When the target nucleic acid contained a plurality of modified nucleobases, even when the target nucleic acid was in an extremely small amount of 0.01 pmol (that is, even when the concentration of the target nucleic acid in the solution was set to 0.1 nM to cause hybridization with the heterogeneous nucleic acid probe and to form the heterogeneous nucleic acid hybrid), clear improvement by the heterogeneous nucleic acid probe over the homogeneous nucleic acid probe was revealed. The experimental conditions on which the improvement was revealed in the present example were (the number of the modified nucleobases in the target nucleic acid = 3, the amount of the target nucleic acid = 0.01 pmol). Meanwhile, the conditions on which the improvement was revealed in Example 3 were (the number of the modified nucleobases in the target nucleic acid = 1, the amount of the target nucleic acid = 0.1 pmol). In other words, even though the number of the modified nucleobase of the present Example was only three times larger than the number of the modified nucleobase of Example 3, detection sensitivity about the amount of the target nucleic acid was increased by as much as an order of 10¹. As described above, when the target nucleic acid contained a plurality of the modified nucleobases, detection sensitivity was amplified, whereby an especially excellent effect of the heterogeneous nucleic acid probe over the homogeneous nucleic acid probe was revealed (Table 8 and FIG. 9).

[Table 8]

**Table 8 Background value and S/N value in measurement of modified nucleobase by nucleic acid probe (3)**

| Nucleic acid probe | Target nucleic acid (DNA) | OD450 | S/N |
|---|---|---|---|
| Homogeneous Backbone: DNA | 1pmol | 0.571 | 2.35 |
| | 0.1pmol | 0.341 | 1.40 |
| | 0.01pmol | 0.272 | 1.12 |
| | 0mol | 0.243 * | - |
| Heterogeneous Backbone: 2'-O-methylated RNA | 1pmol | 0.517 | 7.81 |
| | 0.1pmol | 0.267 | 4.04 |
| | 0.01pmol | 0.140 | 2.12 |
| | 0mol | 0.066 * | - |

| | | | |
|---|---|---|---|
| * Background value | | | |

### Example 5: Use of Heterogeneous Nucleic Acid Probe in Measurement of Modified Nucleobase (4)

The nucleotide sequence of the target nucleic acid (DNA) is 5'-T[C]GTGGTGGACTTCTCTCAATTTTCTAGGGGG-3' (SEQ ID NO:8, [C] indicates 5-methylcytosine), and the nucleotide sequence of the heterogeneous nucleic acid probe for capturing the target nucleic acid is 5'-CCCCCUAGAAAAUUGAGAGAAGUCCACCACAAAAAAAAAA-3' (SEQ ID NO:9, the backbone of the nucleic acid is 2'-O-methylated RNA, the 5'-end is biotin-labeled); those artificially synthesized by Hokkaido System Science Co., Ltd. were used. The heterogeneous nucleic acid probe was designed such that, when a heterogeneous nucleic acid hybrid with the target nucleic acid was formed, the modified nucleobase [C] was present in the single-stranded structure part of the heterogeneous nucleic acid hybrid.

The measurement of the modified nucleobase using the heterogeneous nucleic acid probe was carried out by a method similar to that in Example 1 except that a different target nucleic acid and a different heterogeneous nucleic acid probe were used as described above.

The target nucleic acid was measured by a similar method except that the homogeneous nucleic acid probe (SEQ ID NO:10: 5'-CCCCCTAGAAAATTGAGAGAAGTCCACCACAAAAAAAAAA-3') with DNA as the backbone was used in place of the heterogeneous nucleic acid probe with 2'-O-methylated RNA as the backbone.

As a result of the measurement, even when the target nucleic acid and the heterogeneous nucleic acid probe different from those of Example 1, Example 3, and Example 4 were used, reductions in the background value and increases in the S/N value were revealed (Table 9 and FIGs. 10 and 11). When the amount of the target nucleic acid was 1 pmol or more (that is, when the concentration of the target nucleic acid in the solution was set to 10 nM or more to cause hybridization with the heterogeneous nucleic acid probe and to form the heterogeneous nucleic acid hybrid), an especially excellent effect of the heterogeneous nucleic acid probe over the homogeneous nucleic acid probe was revealed (Table 9 and FIG. 11).

[Table 9]

**Table 9 Background value and S/N value in measurement of modified nucleobase by nucleic acid probe (4)**

| Nucleic acid probe | Target nucleic acid (DNA) | OD450 | S/N |
|---|---|---|---|
| Homogeneous Backbone: DNA | 1pmol | 0.557 | 1.03 |
| | 0.1pmol | 0.568 | 1.05 |
| | 0.01pmol | 0.559 | 1.04 |
| | 0mol | 0.539 * | - |
| Heterogeneous Backbone: 2'-O-methylated RNA | 1pmol | 0.220 | 1.50 |
| | 0.1pmol | 0.167 | 1.14 |
| | 0.01pmol | 0.146 | 0.99 |
| | 0mol | 0.147 * | - |

| | | | |
|---|---|---|---|
| * Background value | | | |

### Example 6: Use of Heterogeneous Nucleic Acid Probe in Measurement of Modified Nucleobase (5)

The nucleotide sequence of the target nucleic acid (DNA) is 5'-GGCTCAGTTTACTAGTGCCATTTGTTCAGTGGTT[C]GT-3' (SEQ ID NO:11, [C] indicates 5-methylcytosine), and the nucleotide sequence of the heterogeneous nucleic acid probe for capturing the target nucleic acid is 5'-CACUGAACAAAUGGCACUAGUAAACUGAGCC-3' (SEQ ID NO:12, the backbone of the nucleic acid is 2'-O-methylated RNA, the 5'-end is biotin-labeled); those artificially synthesized by Hokkaido System Science Co., Ltd. were used. The heterogeneous nucleic acid probe was designed such that, when a heterogeneous nucleic acid hybrid with the target nucleic acid was formed, the modified nucleobase [C] was present in the single-stranded structure part of the heterogeneous nucleic acid hybrid.

The measurement of the modified nucleobase using the heterogeneous nucleic acid probe was carried out by a method similar to that in Example 1 except that a different target nucleic acid and a different heterogeneous nucleic acid probe were used as described above.

The target nucleic acid was measured by a similar method except that the homogeneous nucleic acid probe (SEQ ID NO:13: 5'-CACTGAACAAATGGCACTAGTAAACTGAGCCAAAAAAAAAA-3') with DNA as the backbone was used in place of the heterogeneous nucleic acid probe with 2'-O-methylated RNA as the backbone.

As a result of the measurement, even when the target nucleic acid and the heterogeneous nucleic acid probe different from those of Example 1, Example 3, Example 4, and Example 5 were used, reductions in the background value and increases in the S/N value were revealed (Table 10 and FIGs. 12 and 13). When the amount of the target nucleic acid was 1 pmol or more (that is, when the concentration of the target nucleic acid in the solution was set to 10 nM or more to cause hybridization with the heterogeneous nucleic acid probe and to form the heterogeneous nucleic acid hybrid), an especially excellent effect of the heterogeneous nucleic acid probe over the homogeneous nucleic acid probe was revealed (Table 9 and FIG. 13).

[Table 10]

**Table 10 Background value and S/N value in measurement of modified nucleobase by nucleic acid probe (5)**

| Nucleic acid probe | Target nucleic acid (DNA) | OD450 | S/N |
|---|---|---|---|
| Homogeneous Backbone: DNA | 1pmol | 0.556 | 1.43 |
| | 0.1pmol | 0.409 | 1.05 |
| | 0.01pmol | 0.387 | 1.00 |
| | 0mol | 0.389 * | - |
| Heterogeneous Backbone: 2'-O-methylated RNA | 1pmol | 0.399 | 1.90 |
| | 0.1pmol | 0.242 | 1.15 |
| | 0.01pmol | 0.220 | 1.04 |
| | 0mol | 0.211 * | - |

| | | | |
|---|---|---|---|
| * Background value | | | |

As demonstrated by Examples 1 and 3 to 6, it has been revealed that the present invention is useful for reducing the background value and increase the S/N value regardless of the types of the target nucleic acid and the heterogeneous nucleic acid probe.

### Reference Example 1: Use of Homogeneous Nucleic Acid Probe in Measurement of Modified Nucleobase Containing Modified Nucleobase

The target nucleic acid was measured on the conditions described below. The other experimental conditions (e.g., the volume of a reaction system) were similar to those of Example 1.
The target nucleic acid: DNA containing the modified nucleobase consisting of the nucleotide sequence of SEQ ID NO:1 (10 pmol);
The nucleic acid probe: The homogeneous nucleic acid (DNA) probe consisting of the nucleotide sequence of SEQ ID NO3 (5 pmol); and
The antibody against the modified nucleobase: 100 ng/mL of the anti-methylcytosine antibody (Clone33D3 manufactured by Millipore Corporation).

As a result of the measurement, in the absence of the target nucleic acid (the target nucleic acid (-)), although the detection signal value was high in the presence of both the anti-methylcytosine antibody and the homogeneous nucleic acid probe, reductions in the background value of the detection signal were revealed in the absence of either the anti-methylcytosine antibody or the homogeneous nucleic acid probe (Table 6). These facts suggest that the high detection signal value measured in the presence of both the anti-methylcytosine antibody and the homogeneous nucleic acid probe is caused by their non-specific binding.

[Table 11]

**Table 11 Signal value measured by measurement of modified nucleobase by homogeneous nucleic acid probe**

| Antibody against modified nucleobase | Homogeneous nucleic acid (DNA) probe | Target nucleic acid (DNA) | OD450 |
|---|---|---|---|
| + | + | + | 3.082 |
| | | - | 0.960 |
| - | + | + | 0.142 |
| | | - | 0.141 |
| + | - | + | 0.125 |
| | | - | 0.106 |
| - | - | + | 0.087 |
| | | - | 0.095 |

From the foregoing, it has been revealed that the non-specific binding of the antibody and the homogeneous nucleic acid probe can cause non-specific increases in the background value of the detection signal.

### Industrial Applicability

The method and kit of the present invention are useful for measuring a modified nucleobase.

## Claims

1. A method for measuring a modified nucleobase, the method comprising:
(1) incubating a nucleic acid sample and a heterogeneous nucleic acid probe in a solution; and
(2) measuring the modified nucleobase using an antibody against the modified nucleobase in the solution obtained at (1).

2. The method according to claim 1, wherein the nucleic acid sample contains a target nucleic acid containing the modified nucleobase, and the steps (1) and (2) are performed by (1') and (2'), respectively:
(1') reacting the nucleic acid sample containing the target nucleic acid containing the modified nucleobase and the heterogeneous nucleic acid probe in the solution by incubation to form a heterogeneous nucleic acid hybrid composed of the target nucleic acid and the heterogeneous nucleic acid probe; and
(2') measuring the modified nucleobase using the antibody against the modified nucleobase in a solution containing the heterogeneous nucleic acid hybrid.

3. The method according to claim 1 or 2, wherein the target nucleic acid is a target nucleic acid potentially containing two or more modified nucleobases.

4. The method according to any one of claims 1 to 3, further comprising adding the heterogeneous nucleic acid probe to a solution containing the nucleic acid sample to prepare a solution containing both the nucleic acid sample and the heterogeneous nucleic acid probe.

5. The method according to any one of claims 1 to 4, wherein the nucleic acid sample is a sample containing a target DNA containing the modified nucleobase.

6. The method according to any one of claims 1 to 5, wherein the heterogeneous nucleic acid probe is an RNA probe.

7. The method according to any one of claims 1 to 6, wherein a nucleobase that composes the modified nucleobase is cytosine.

8. The method according to any one of claims 1 to 7, wherein the modified nucleobase is methylcytosine.

9. The method according to any one of claims 2 to 8, wherein the heterogeneous nucleic acid probe is designed such that an unpaired part of the modified nucleobase is formed in a double-stranded structure part of the heterogeneous nucleic acid hybrid.

10. The method according to any one of claims 2 to 9, wherein the heterogeneous nucleic acid probe is designed such that the modified nucleobase is present in a single-stranded structure part of the heterogeneous nucleic acid hybrid.

11. The method according to any one of claims 1 to 10, wherein the measurement of the modified nucleobase using the antibody against the modified nucleobase is performed by ELISA.

12. A kit for measuring a modified nucleobase, the kit comprising:
(I) a heterogeneous nucleic acid probe; and
(II) an antibody against the modified nucleobase.
